# EUROPEAN PATENT APPLICATION

(11) **EP 1 688 503 A1**
(43) Date of publication of application: **09.08.2006**
(21) Application number: 04792285.1
(22) Date of filing: 13.10.2004
(51) Int. Cl.: C12Q 1/02, G01N 33/48

(54) **METHOD OF TREATING CELLS**

(30) Priority: 20.10.2003 JP 2003359336
(71) Applicant: SYSMEX CORPORATION, Kobe-shi, Hyogo 651-0073 (JP)
(72) Inventor: NAKANO, Koichi, c/o Sysmex Corporation, Kobe-shi Hyogo 6510073 (JP); UMETSU, Ayako, Odate-shi, Akita 0170046 (JP); OOI, Yuko, Yokohama-shi, Kanagawa 241-0817 (JP)
(74) Representative: HOFFMANN EITLE
(86) International application number: PCT/JP2004/015041
(87) International publication number: WO 2005/038044

(57) **Abstract**

The invention provides methods of treating cells wherein cells in a specimen containing mucus-producing cells are stabilized, mucus in the specimen is removed without affecting the cell morphology, or cells in the specimen are dispersed individually; and reagent kits to be used in said treating methods. After the cells are stabilized by treatment with a solution containing an aldehyde compound, the cell populations can be dispersed by treatment with protease. In the case of specimens containing cells aggregated with mucus, the processes of stabilizing and treating with protease may be conducted after the mucus is removed. In order to remove mucus, cysteine and/or a compound derived therefrom is preferably used.

## Description

### Technical Field

The present invention relates to methods of treating cells wherein specimens containing cells collected from the body are appropriately processed for cytodiagnosis or flow cytometry, and treatment reagent kits used for the methods.

### Background Art

As a screening method for early detection of cervical adenocarcinoma, cytodiagnosis is utilized effectively in medical examinations.

Cytodiagnosis for cervical adenocarcinoma is performed by scraping cells from cervical surface with a cotton swab or a scraper, etc., immediately smearing the scraped cells on a slide glass to prepare a sample and observing the sample under a microscope or the like. In medical institutions where it is necessary to treat a large amount of samples in medical examinations, cell populations collected by scrape are generally preserved in preservative solutions containing alcohol (e.g. patent reference 1) and transferred to laboratories where cells are smeared on slide glasses for Papanicolaou staining, followed by microscopic examination. Then, the presence of cancer cells is determined by morphology of clusters of cells.

In either way, a lot of time and handling processes are required to prepare smears for each sample and to examine them under microscopes. Therefore, the demand for automation of cytodiagnosis has increased in recent years.

The methods for automation of cytodiagnosis include flow cytometry which uses immunocytochemistry. In the immunocytochemistry-based flow cytometry, distribution of size, DNA content and expression level of membrane antigens of individual cells in a cell population are determined by staining a protein marker expressed on cells with a fluorescent-labeled antibody, suspending cells in a liquid, irradiating the suspension with laser light and measuring fluorescent light emitted by individual cells. The presence and the amount of cancer cells may also be determined by using a specific antibody for cancer cells to be detected. To increase the accuracy of determination in automation of cytodiagnosis, each cancer cell must be stained with the antibody specifically. Moreover, in order to decide whether a cell is a cancer cell or not by the cell morphology and to count the number of cancer cells, cells have to be dispersed individually with the cell morphology not being affected.

Cells collected by scraping uterine cervix comprise two kinds of cells, squamous cells and glandular cells, and most of the cells collected are squamous cells. Uterine corpus at the back of the cervix is mainly comprised of glandular cells secreting mucus, therefore, cells collected from uterine cervix are in a condition of aggregation covered with mucus produced in uterine corpus. Such specimens in which cells are aggregated with glue of mucus may not be directly analyzed by flow cytometer. Moreover, in performing antibody staining, an antibody is adsorbed nonspecifically to components of mucus, which introduces detection noise, therefore accurate cytodiagnosis may not be expected. When such specimen where cells aggregate with mucus is analyzed by flow cytometry, it is required that the mucus is removed and the cells are separated to individual cells such that a labeled antibody can specifically react with the individual cell surfaces without mucus.

A method to remove mucus for cytodiagnosis is described in the patent reference 2. In the method, mucus is dissolved and cells are dispersed by adding a sputum specimen into 30% ethanol - PBS solution containing 0.1 to 0.2% methyl cysteine for reaction. In a sputum specimen, cells exfoliated from lung are suspended in a viscous fluid, therefore individual cells can be obtained by dissolving mucus in sputum. However, in a case of clusters of cells aggregated with mucus like glue, such as the cell populations derived from uterine cervix, mucus can not be dissolved sufficiently even with the above-mentnioned mucus dissolving solution, and there still remains some noise caused by nonspecific adsorption of antibodies. Furthermore, it is not possible to disperse cells so that they can be analyzed by flow cytometry.

On the other hand, a cell preservative solution disclosed in the patent reference 1 (U.S. Pat. No. 5,256,571) comprises a chelating agent such as ethylenediamine tetraacetic acid (EDTA) in alcohol. A chelating agent is known as having an effect on preventing cells from clumping. However, in the case that a specimen where cells are aggregated with glue of mucus, such as the one from uterine cervix is preserved in the above preservative solution, the cells can not be separated individually and the mucus can not be dissolved. Consequently, it fails to decrease nonspecific adsorption of a labeled antibody.

The patent reference 2 discloses a liquid for protecting cells, which is used as a tissue-washing liquid, an artificial cerebrospinal fluid, an intraocular perfusate, etc. This cell-protecting liquid is an electrolyte solution containing N-acetylcysteine and/or N-diacetylcystine at concentrations of 0.1 to 10 mM (about 0.0016% to 0.16%). A cell fixation and preservation solution comprising 0.1 to 0.2 mass% of methyl cysteine as a mucus dissolving agent is disclosed in the patent reference 3 and a cell fixation and preservation solution comprising 0.1 to 0.2 mass% of methyl cysteine as a mucus dissolving agent in a buffer solution comprising ethanol, sodium chloride, sucrose or propylene glycol is disclosed in the patent reference 4.

However, any preservative solutions for cells disclosed can not sufficiently dissolve mucus in the clusters of cells attached together with mucus, such as cell populations collected by scraping uterine cervix, and as a result, cells in the cluster can not be dispersed to individual cells and nonspecific adsorption of labeled antibodies to mucus can not be avoided.

On the other hand, it has been known that protease such as trypsin is used in the method to disperse attached or aggregated cells to individual cells. However, the dispersion of cells with protease results in not only separation of cells but also dissolution of cell membranes, therefore, the method is not suitable for preparing specimens for diagnosis to decide whether a cell is a tumor cell or not by images or data concerning cell morphology, or for immunocytochemistry-based flow cytometry in which antibody is bound to surfaces of cell membranes. Furthermore, fixation of cells by cell fixative and preservative solutions disclosed in the above patent references 1 to 4 is insufficient to prevent cell membranes from being digested by protease.

Besides, if a cell specimen is acted on proteases directly without removing mucus from the cell specimen in which cells are aggregated together with mucus, such as a specimen of cell populations collected by scraping the surface of uterine cervix, it takes too much time to separate individual cells, because proteases have to work first for dissolving mucus before dispersing cells individually. On the other hand, if the protease concentration is increased or the enzyme reaction time is set to be long, a protease may also digest cell membranes after removing mucus. Accordingly, it is difficult to establish and control an appropriate condition in which individual cells are dispersed by only removing mucus on cell surfaces and the cell morphology is not affected.

Patent reference 1: U.S. Pat. No. 5,256,571
Patent reference 2: JP Unexamined Patent Publication No. Hei 10-323183
Patent reference 3: JP Examined Patent Publication No. Hei 7-46101
Patent reference 4: JP Examined Patent Publication No. Hei 7-46100

### Disclosure of the Invention

### Problems to be resolved by the invention

The present invention was made in view of the circumstances described above. One object of the present invention is to provide methods of treating cells in order that mucus is removed without affecting the morphology of cells and then cells are dispersed individually in specimens containing cells secreting mucus, such as those derived from uterine cervix, and reagent kits used in said methods of treating cells.
It is another object of the present invention to provide methods of stabilizing specimens containing cells secreting mucus.
It is a further object of the present invention to provide methods of treating cells aggregated in specimen to disperse cells individually without affecting the cell morphology and cell treatment reagent kits used therefor.

### Means of Solving the Problems

A first method of treating cells of the present invention comprises the steps of removing mucus from a specimen containing cells and the mucus and treating the specimen with a solution containing an aldehyde compound to stabilize the cells. The treating step follows the removing step.

A second method of treating cells of the invention comprises the step of treating the specimen with a protease after stabilizing cells in the specimen, in addition to the steps recited in the first method. That is to say, the second method of treating cells comprises the steps of removing mucus from a specimen containing cells and the mucus, treating the specimen with a solution containing an aldehyde compound to stabilize the cells after the removing step, and treating the specimen with a protease after stabilizing cells in the specimen.

A third method of treating cells of the present invention comprises the steps of treating a specimen containing cells with a solution containing an aldehyde compound to stabilize the cells, and treating the cells with a protease after stabilizing cells in the specimen. The cells contained in the specimen are usually not adhered by mucus but may comprise a cell adhered to by mucus.

In the first, second, and third methods, said step of removing mucus is preferably performed by treating the specimen containing cells and mucus with a solution containing cysteine and/or a compound derived therefrom. The cells contained in the specimen may comprise a uterin cervix cell. The cells applied to any of said treating methods may comprise a cell preserved in a solution containing alcohol. In said methods of treating cells, it is preferred that the aldehyde compound is at least one selected from the group consisting of paraformaldehyde, formaldehyde, and glutaraldehyde. Furthermore, collagenase is preferably used as the protease in said methods.

A method of preparing a sample for flow cytometry of the present invention comprises preparing a sample for flow cytometry after conducting of stabilizing cells in the specimen and treating the specimen with a protease.

An inventive reagent kit of treating a specimen suitable for said first method of treating cells comprises a first reagent containing cysteine and/or a compound derived therefrom and a second reagent containing an aldehyde compound.

An inventive reagent kit of treating a specimen suitable for said second method of treating cells comprises a first reagent containing cysteine and/or a compound derived therefrom, and a second reagent containing an aldehyde compound, and a third reagent containing a protease.

An inventive reagent kit of treating a specimen suitable for said third method of treating cells comprises a first reagent containing an aldehyde compound, and a second reagent containing a protease.

In said reagent kits of the present invention, collagenase is preferably used as the protease, and the preferable content of collagenase is in the range of 0.1 to 0.5 mass%. The preferable aldehyde compound may be at least one selected from the group consisting of paraformaldehyde, formaldehyde, and gultalaldehyde.

### Effect of the Invention

According to the methods of treating cells of the present invention, since cells are exposed to protease after stabilized, the cells may be dispersed efficiently while digestion of cells themselves by the protease may be suppressed even in specimens with cells being aggregated. Especially in the case of clusters of cells, which are aggregated with mucus, cells can be dispersed more effectively by removing mucus and then stabilizing cells without mucus.
The treatment reagent kits of the present invention are used suitably for performing the methods of treating cells of the present invention.

### Brief Description of the Drawings

Fig. 1 is a micrograph (×400) obtained in Example 1.
Fig.2 is a micrograph (×400) obtained in Referential Example 1.
Fig.3 is a micrograph (×400) obtained in Comparative Example 1.
Fig.4 is a micrograph (×400) obtained in Comparative Example 2
Fig.5 is a micrograph (×400) obtained in Referential Example 2.
Fig.6 is a micrograph (×100) obtained in Example 2.
Fig.7 is a micrograph (×100) obtained in Example 3.
Fig.8 is a micrograph (×100) obtained in Example 4.
Fig.9 is a micrograph (×100) obtained in Example 5.
Fig.10 is a micrograph (×100) obtained in Example 6.
Fig.11 is a micrograph (×100) obtained in Example 7.
Fig.12 is a micrograph (×600) obtained in Example 7.
Fig. 13 is a micrograph (transmission image; ×600) obtained in Example 7.

### Best Mode for Carrying Out the Invention

In advance of Lhe description of the methods of treating cells of the present invention, a treatment reagent kit to stabilize and fix specimens and a reagent kit for treatment to disperse cells are described in the below.

The reagent kit of the present invention to stabilize and fix specimens containing cells with mucus attached comprises a combination of a first reagent kit containing cysteine and/or a compound derived therefrom and a second reagent kit containing an aldehyde compound.

The first reagent containing cysteine and/or a compound derived therefrom is mainly used for removing mucus in specimens of cell populations, in which mucus-producing cells are aggregated together with mucus, such as specimens of cell populations collected by scraping the surface of uterine cervix. More specifically, cysteine and/or a compound derived therefrom can remove mucus easily in washing operations by breaking disulfide bonds of mucus existed on cell surfaces, denaturing the secondary structures of mucus and decreasing the viscosity of mucus.

The first reagent comprises water, saline, buffers such as PBS (phosphate buffered saline) and Tris as a solvent for cysteine and/or a compound derived therefrom.

Examples of cysteine and/or compounds derived therefrom include methyl cysteine, acetyl cysteine, L-cysteine and the like.
The concentration of cysteine and/or a compound derived therefrom in the first reagent is preferably 5 mass% or above, more preferably 10 to 20 mass%. Because mucus can not be removed sufficiently with the first reagent containing less than 5 mass% of cysteine and/or a compound derived therefrom.

The second reagent containing an aldehyde compound works for stabilizing cells where the digestive activity of protease becomes weak against cells after removing mucus from their surfaces. Aldehyde groups in aldehyde compound react with the amino terminus of proteins to form cross-bridge structures, thereby the proteins change their secondary or tertiary structure to become more difficult to be decomposed by protease.

For the aldehyde compound in the second reagent, paraformaldehyde, formaldehyde, glutaraldehyde or a mixture of those aldehydes may be used.

Solvents for aldehyde compounds in the second reagent may be solvents which do not react with aldehyde groups. Preferable solvent is water such as distilled water, ion-exchange water and purified water.
Other component which may preferably be contained in the second reagent is about from 0.1 to 0.4 mass% picric acid. Picric acid may enhance tissue permeability of aldehyde compounds.

According to the present invention, the reagent kit for the treatment to disperse cells in specimens containing cells with mucus attached comprises, in addition to the combination of the first reagent and the second reagent, which are comprised for cell stabilization, a third reagent containing a protease for separating cells individually.

Examples of the protease include trypsin, pronase, pepsin, elastase, collagenase and the like. They may be used individually or in a combination of more than two kinds of proteases.
Among these proteases, collagenase is used preferably. When a protease with weak digestive power is used, the time required for dispersion can be determined within relatively wide range, therefore the difference of appropriate digestion time required for each specimen is not problematic. Kinds of collagenases are not particularly limited, and the collagenases may be derived from animals or bacteria, and the substrate specificities of collagenases derived from animals are not particularly limited.

Solvents in the third reagent may be solvents which do not denature proteases. It is preferable to use water such as distilled water, ion-exchange water and purified water for the solvent.

The concentration of the protease in the third reagent is appropriately decided according to the kind of protease.
In the case of a protease which has high power of digestion, such as trypsin, the concentration of the protease is preferably about 0.05 to 0.1 mass %, and in the case of collagenase, the preferable concentration is about 0.1 to 1.0 mass %.

The reagent kits of the present invention may be used for stabilization and dispersion not only for specimens containing cells and mucus but also for specimens which do not need treatment of removing mucus. The reagent kit for performing treatment to disperse specimens of cell populations which do not need to remove mucus comprises a first reagent containing an aldehyde compound for stabilization (corresponding to the second reagent in the above reagent kit for treating specimens containing cells with mucus attached) and a second reagent containing a protease for dispersion (corresponding to the third reagent in the above reagent kit to treat specimens containing cells with mucus attached).

The methods of treating cells of the present invention are described below.
The methods of treating cells of the present invention are suitable for preparing specimens for smear analysis or flow cytometry from specimens such as cells aggregated together. The method comprises a process of stabilization and a process of dispersion.

The process of stabilization is to stabilize cell membranes by using the reagent containing an aldehyde compound. Cross-linking by the aldehyde compound with cytoskeletal protein may contribute to weakening digestion by protease.

The time of reaction with aldehydes is determined depending on the kind of aldehyde compound, however, 10 to 30 minutes is preferably employed in general. The reaction time less than one minute is not enough for stabilization, and the reaction for over 1 hour allows too much cross-linking and enzymatic digestion of mucus cannot be performed. When the reaction is conducted at room temperature, the time of reaction is appropriately from 10 minutes to 30 minutes, however, when the reaction is performed at 4°C, the rate of reaction is slow and the reaction time can be up to 12 hours. The optimal concentration of the aldehyde compound is 2% to 10%. Generally, when the reaction is performed at a low temperature and a low concentration, the reaction time can be long.
In the process of stabilization, two or three times repetition of a series of procedures of reaction with a reagent containing an aldehyde compound and washing of cells is preferable.

The process of dispersion is a process to disperse clusters of cells aggregated by protease contained in the reagent for treatment of dispersion in the reagent kits of the present invention.

The time of digestion by protease is appropriately selected according to the kind and the concentration of protease used. In general, when a protease with strong power of digestion such as trypsin and pepsin are used at 0.01 to 0.05 mass%, the time of digestion is about 20 minutes. On the other hand, when collagenase with weak digestion power is used at 0.1 mass%, the time is about 0.5 to 90 minutes, and when pronase is used at 0.05 mass%, the time of digestion is about 30 to 90 minutes.

In the case of collagenase of which digestion power is weak, the range of the appropriate time of digestion is wide, therefore, even if there are differences of appropriate times of digestion according to specimens, a certain period of time for digestion may be determined. On the other hand, in the case of protease with strong power of digestion such as trypsin and pepsin, the range of the appropriate time of digestion is narrow, therefore, if a certain period of time is established, a problematic situation may occur in which cells are not dispersed enough in some specimens and too much digestion proceeds to lead destruction of the cell morphology in other specimens.

After being digested, cells are separated in centrifugations, etc. and collected, to obtain specimens in which cells are dispersed with the cell morphology being maintained. The dispersed cell specimens are stained by fluorescence-labeled antibody to prepare samples for flow cytometry. Cell samples to be examined may be prepared by staining or other ways according to the kind of detection methods.

The specimen targeted by the method of treating cells of the present invention may be either specimens immediately after collection or specimens preserved in a preservative solution containing alcohol (e.g. PreservCyt® available from Cytyc Corporation).
Preferable examples of the preservative solution containing alcohol include preservative solutions containing 30 to 60 mass% lower alcohol, such as methanol, ethanol, propanol and isopropanol.

Specimens containing cells with mucus attached may be taken from the preservative solution containing alcohol and subjected directly to the above stabilization and dispersion processes. However, it is more preferable to remove mucus before the stabilization process.

Any procedures for removing mucus can be employed as far as mucus contained in specimens is removed without affecting the cell morphology. Examples of such procedures for removing mucus include dissolving mucus by decreasing its viscosity. An appropriate procedure may be selected according to the kind of specimen.

In the case that cells are aggregated together with mucus which is like glue, such as cells derived from uterine cervix, it is preferable to use a treatment solution containing cysteine and/or a compound derived therefrom, which is the first reagent used in the above reagent kit for stabilization and the reagent kit for dispersion of specimens containing cells with mucus attached. Mucus is dissolved in the reaction with cysteine and/or a compound derived therefrom to be removed easily. After the reaction with cysteine and/or a compound derived therefrom, cells are separated with a centrifuge, etc. and mucus is removed. In the case that cells with mucus being thus removed are provided for the stabilization process, nonspecific adsorption of antibodies used for antibody staining may be prevented and the treatment time may be reduced, as compared with the case in which specimens containing cells with mucus attached are directly provided for the stabilization process.

When treatment for removing mucus is performed, it is important to conduct a stabilization process after removing mucus. Because in stabilization by cross-linking, not only cells are stabilized, but also mucus is cross-linked and stabilized, therefore, if treatment with cysteine and/or a compound derived therefrom is performed after stabilization, the process of removing mucus by using cysteine and/or a compound derived therefrom is not achieved any more. On the other hand, if stabilization is performed after mucus is removed, the second reagent containing an aldehyde compound can react with cells directly and therefore stabilization of cells is achieved with the second reagent at low concentrations for a short time.

The process of removing mucus may be performed during preservation of the specimens. Removing mucus during preservation of the specimen can be achieved by preserving a specimen in a preservative solution such as an aqueous solution containing 30 to 60 mass% alcohol and cysteine and/or a compound derived therefrom.

### Examples

### [Stabilization effect of aldehyde compound]

### Example 1:

After cell populations of uterine cervix obtained by scraping the surface of uterine cervix were fixed and stored in PreservCyt® solution (Cytyc Corporation, USA), they were transferred to a 1.5 ml centrifuge tube (about 3 x 10⁴ cells/sample) and the cells were separated by centrifugation (10000 rpm; 1 minute; 4°C) and the supernatant was removed.

To the cell populations of uterine cervix thus collected was added 1 ml of a mixture of 0.1% Tween 20 (Sigma) and 0.001M phosphate buffer (Sigma; pH7.4) (hereinafter referred to as PBS-T) and the cells were resuspended. The cells were collected by another centrifugation (10000 rpm; 1 minute; 4°C).

The collected cells were suspended in Zamboni solution and shaken on a rotary shaker at 25°C for 15 minutes. Zamboni solution is a mixture of A solution (saturated aqueous picric acid (0.67% picric acid solution)) and B solution (20% aqueous paraformaldehyde) and it is prepared by mixing A solution, B solution and distilled water in a ratio of 3:2:15.
After rotary shaking, cells were collected by separation in centrifugation (10000 rpm; 1 minute; 4°C), and removing the supernatant that was the reactant solution.

To the cells thus collected was added water containing 0.1 mass% collagenase (type II) (Example 1) and the reaction was performed at 37°C for 40 minutes. The digestion reaction by protease was stopped by adding 1200µl of protease inhibitor cocktail (P8340 (Sigma) used at a dilution of 1:100 with TBST) at 4°C.
After the digestion reaction was stopped, the cells were collected by centrifuging (10000 rpm; 1 minute; 4°C) for cell separation and removing the supernatant of resultant.

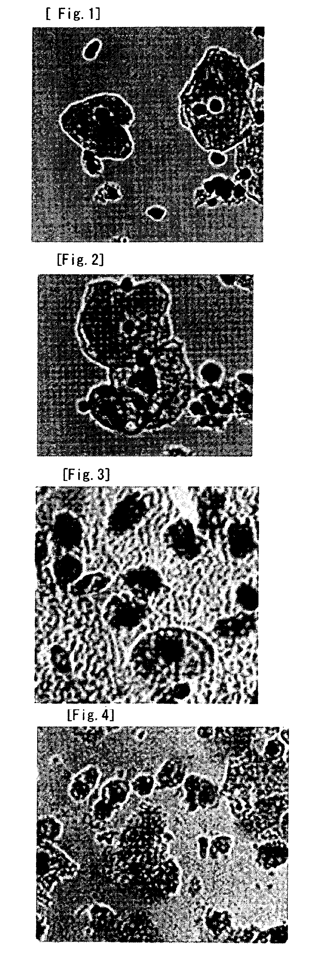

The cells were stained with 5% Giemsa Stain Solution (Sysmex) for 15 minutes, and then washed thoroughly with PBS, and observed under a light microscope to give a micrograph of Fig. 1.

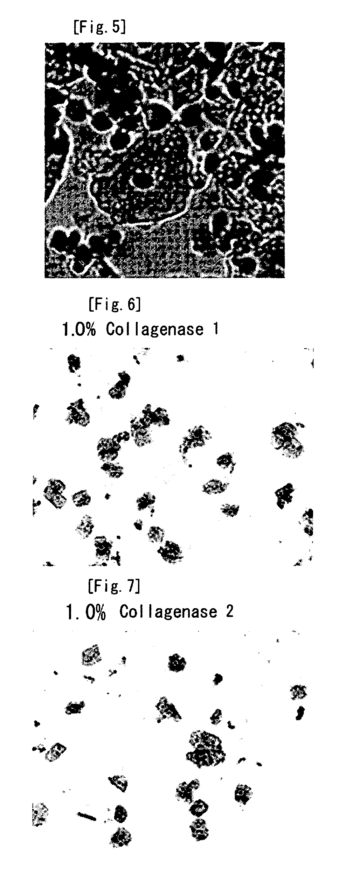

For comparison, the cells without being subjected to digestion reaction by protease (Referential Example 1) were stained in the same manner as above and observed under a light microscope to give a micrograph of Fig. 2.

In comparison of Fig. 1 and Fig. 2, it is understood that clusters of cells are dispersed to individual cells with the cell morphology being maintained in Example 1.

### Comparative Examples 1 and 2:

According to the Example 1 except that the operation for stabilization by Zamboni solution was not conducted, the cells were collected and stained with 5% Giemsa Stain Solution (Sysmex) for 15 minutes, and then washed thoroughly with PBS, and observed under a light microscope to give micrographs shown in Fig. 3 and Fig. 4.

For comparison, the cells without being subjected to digestion reaction by proteases (Referential Example 2) was stained in the same manner as above and observed under a light microscope to give a micrograph of Fig. 5.

In Figs 3 and 4, the cell membranes were dissolved and bare nuclei were swollen. It was revealed that when stabilization was not performed by an aldehyde compound, proteases not only dispersed cell populations to individual cells but also digested cell membranes.

### [Kinds of proteases and those dispersing effect]

### Examples 2 to 6:

Digestion reactions of Examples 2 to 6 were performed according to Example 1. Fur these examples, protease solutions used in table 1 and digestion times shown in table 1 were applied. Collagenase type I and collagenase type II have different proteolytic activities for specific sites of protein. Collagenase type II has higher clostripain activity.

**Table 1**

| Example No. | Protease solution | Digestion Time (minutes) |
|---|---|---|
| 2 | 1. 0% collagenase (type I) | 8 0 |
| 3 | 1. 0% collagenase (type II) | 8 0 |
| 4 | 0. 1% pronase | 8 0 |
| 5 | 0. 1% trypsin | 4 0 |
| 6 | 0. 01% elastase | 8 0 |

After the reaction finished, staining was performed in the same manner as Example 1 and observation was conducted under a light microscope. The micrographs obtained are in Fig. 6 to Fig. 10.

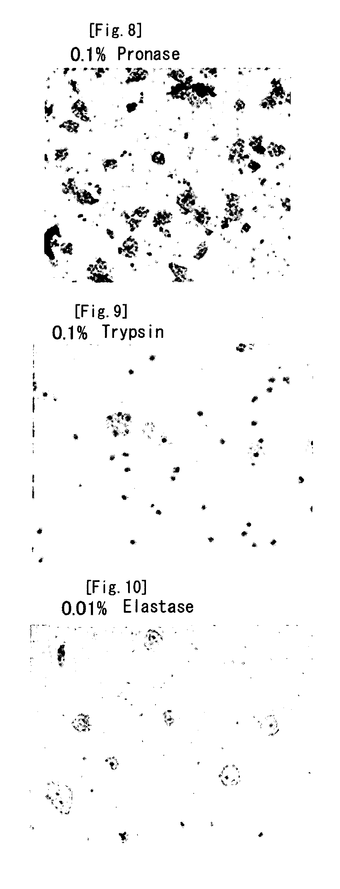

The cells digested by collagenases (Fig. 6 and Fig. 7) and by pronase (Fig. 8) were dispersed sufficiently with the cell morphology being maintained well. By comparing Example 1 with Examples 2 and 3, it is understood that cells can be dispersed with the cell morphology being maintained regardless of the concentration of protease and/or digestion time.
On the other hand, when trypsin (Fig. 9) and elastase (Fig. 10) were used, bare nuclei were observed, which revealed that cell membranes were digested. It is found that these proteases have strong digestion power, therefore, the determination of the concentration of protease solutions and the reaction time largely affects maintenance of morphology.

### [Effect of removing mucus]

### Example 7:

Uterine cervix cells containing glandular cells were used for specimens. After uterine cervix cells obtained by scraping the surface of uterine cervix were fixed and stored in a preservative solution (PreservCyt® manufactured by Cytyc Corporation), they were transferred to a 1.5 ml centrifuge tube (about 3 x 10⁴ cells/sample) and cells were separated by centrifugation (10000 rpm; 1 minute; 4°C) and the supernatant was removed.

To the collected cells was added 1 ml of PBS-T (0.1% Tween 20 (Sigma)+0.001M phosphate buffer (Sigma; pH7.4) ) and the cells were resuspended.

The cells were washed by another centrifugation (10000 rpm, 1 minute, 4°C) for cell separation, removal of supernatant and cell collection. The cells washed were resuspended in 500 µl of a preservative solution.

To the cell suspension thus obtained was added 500µl of 10% N-acetyl-L-cysteine (hereinafter referred to as AcCys) solution and the mixture was reacted at ambient temperature for one minute.
Another 500 µl of a preservative solution was added to stop the reaction of dissolving mucus. After the mucus dissolving reaction, centrifugation (10000 rpm; 1 minute; 4°C) was performed to separate cells, the supernatant containing resultant was removed and the cells after removal of mucus were collected.

The cells collected were washed by resuspension in 1 ml of PBS-T, another centrifugation (10000 rpm; 1 minute, 4°C) for cell separation, and removal of the supernatant. This washing procedure was repeated two times.
The collected cells were suspended in Zamboni solution and shaken on a rotary shaker at 25°C for 15 minutes.
After rotary shaking, cells were separated by centrifugation (10000 rpm, 1 minute, 4°C), the supernatant was removed and the cells were collected.

To cells thus collected was added water containing 0.1 mass% collagenase (type I and type II) and the digestion reaction was performed at 37°C for one minute. The reaction was stopped by adding 1200 µl of protease inhibitor cocktail (P8340 (Sigma) used at a dilution of 1:100 with TBST) at 4°C.
After the digestion reaction was stopped, the cells were collected by centrifuging (10000 rpm; 1 minute; 4°C) for cell separation and removing the supernatant containing resultant.

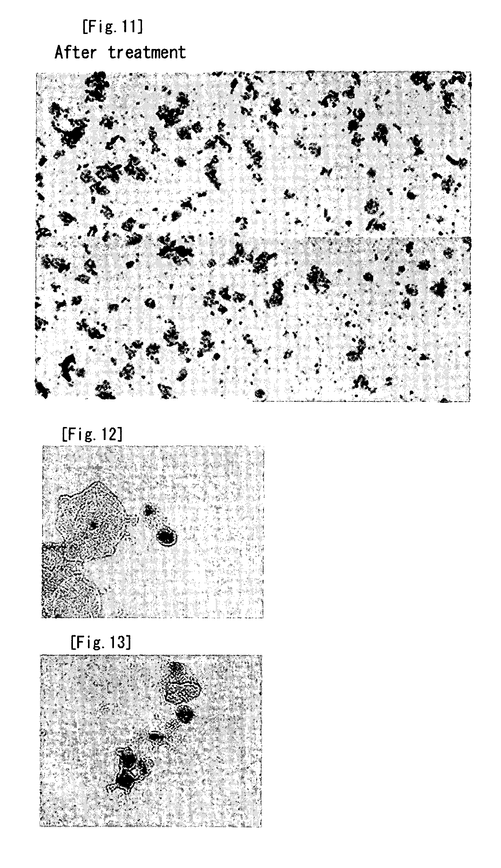

The cells were divided into two groups. The cells in one group were stained with 5% Giemsa Stain Solution (Sysmex) for 15 minutes, and then washed thoroughly with PBS, and observed under a light microscope to give micrographs of Fig. 11 (×100) and Fig. 12 (×600). For the cells in another group, NMP179 antibody labeled with alkaline phosphatase was used. NMP179 antibody binds to the cells derived from uterine cervix except for normal squamous cells. The antibody can bind to the cells such as squamous carcinoma, glandular carcinoma or normal glandular cells. For colorimetric substrate, Vector Red (Vector Red Alkaline Phosphatase Substrate Kit I Cat.No.SK-5100 available from Vector Laboratories, Inc.) was used. By observing under a microscope, a micrograph of Fig. 13 was obtained.

Fig. 11 and Fig. 12 showed good dispersion of both squamous cells and glandular cells. Accordingly, it was confirmed that dispersion using a protease was effective even in a short time reaction of one minute when the operation of removing mucus was performed before cell fixation.
Fig. 13 showed the part of cancer cells which reacted specifically with the antibody (black part). It was confirmed that the dispersed glandular cells were stained with NMP179 antibody, and even after the dispersion by enzyme reaction, the cell morphology was maintained.

### Industrial Applicability

The methods of treating cells of the present invention can be used for specimens in which cells are aggregated and even for specimens of cell populations aggregated with mucus like glue to disperse cells individually without affecting the cell morphology. Therefore, the methods can be used as pretreatment for cytodiagnosis of cell clusters aggregated with mucus, such as cell populations derived from uterine cervix.

The reagent kits of the present invention are combinations of reagents which effect the methods of treating cells of the present invention, and the reagent kits enable the methods of treating cells of the present invention to be performed easily.

## Claims

1. A method of treating cells comprising the steps of:
removing mucus from a specimen containing cells and the mucus; and
after the removing step, treating the specimen with a solution containing an aldehyde compound to stabilize the cells.

2. The method of treating cells according to claim 1, further comprising the step of treating the specimen with a protease after stabilizing cells in the specimen.

3. The method of treating cells according to claim 1, wherein said removing step is conducted by treating the specimen with a solution containing cysteine and/or a compound derived therefrom.

4. A method of treating cells comprising the steps of:
treating a specimen containing cells with a solution containing an aldehyde compound to stabilize the cells; and
treating the specimen with a protease after stabilizing cells in the specimen.

5. The method of treating cells according to claim 4, wherein the cells comprise cells adhered to by mucus.

6. The method of treating cells according to claim 1, wherein the cells comprise a uterin cervix cell.

7. The method of treating cells according to claim 5, wherein the cells comprise a uterin cervix cell.

8. The method of treating cells according to claim 1, wherein the cells comprise a cell preserved in an alcohol solution.

9. The method of treating cells according to claim 4, wherein the cells comprise a cell preserved in an alcohol solution.

10. The method of treating cells according to claim 1, wherein the aldehyde compound is at least one selected from the group consisting of paraformaldehyde, formaldehyde, and glutaraldehyde.

11. The method of treating cells according to claim 4, wherein the aldehyde compound is at least one selected from the group consisting of paraformaldehyde, formaldehyde, and glutaraldehyde.

12. The method of treating cells according to claim 2, wherein the protease is collagenase.

13. The method of treating cells according to claim 4, wherein the protease is collagenase.

14. A method of preparing a sample for flow cytometry comprising a step of preparing a sample for flow cytometry after conducting the method of treating cells according to claim 2.

15. A method of preparing a sample for flow cytometry comprising a step of preparing a sample for flow cytometry after conducting the method of treating cells according to claim 4.

16. A reagent kit of treating a specimen containing cells and mucus comprising:
a first reagent containing cysteine and/or a compound derived therefrom; and
a second reagent containing an aldehyde compound.

17. The kit according to claim 16, further comprising a third reagent containing a protease.

18. A reagent kit of treating a specimen containing cells and mucus comprising:
a first reagent containing cysteine and/or a compound derived therefrom; and
a second reagent containing a protease.

19. The kit according to claim 17, wherein the protease is collagenase and the concentration of the collagenase falls in the range of 0.1 to 0.5 mass%.

20. The kit according to claim 18, wherein the protease is collagenase and the concentration of the collagenase falls in the range of 0.1 to 0.5 mass%.

21. The kit according to claim 16, wherein the aldehyde compound is at least one selected from the group consisting of paraformaldehyde, formaldehyde, and gultalaldehyde.

22. The kit according to claim 18, wherein the aldehyde compound is at least one selected from the group consisting of paraformaldehyde, formaldehyde, and gultalaldehyde.
